(19) Europäisches Patentamt

European Patent Office

Office européen des brevets

(11) EP 1 215 199 A1

(12) EUROPEAN PATENT APPLICATION

(43) Date of publication:
19.06.2002 Bulletin 2002/25

(51) Int Cl.⁷: **C07C 233/36**, C07C 323/60, C07H 21/04, H01B 1/12, H01L 51/20, H01L 51/30, G06N 3/12

(21) Application number: 00126966.1

(22) Date of filing: 08.12.2000

(84) Designated Contracting States:
AT BE CH CY DE DK ES FI FR GB GR IE IT LI LU MC NL PT SE TR
Designated Extension States:
AL LT LV MK RO SI

(71) Applicant: Sony International (Europe) GmbH
10785 Berlin (DE)

(72) Inventors:
• Ford, William E., c/o Adv. Techn. Ctr. Stuttgart
70327 Stuttgart (DE)
• Wessels, Jurina, c/o Adv. Techn. Ctr. Stuttgart
70327 Stuttgart (DE)
• Yasuda, Akio, c/o Adv. Techn. Ctr. Stuttgart
70327 Stuttgart (DE)

(74) Representative: Schohe, Stefan
Forrester & Boehmert
Pettenkoferstrasse 20-22
80336 München (DE)

(54) **Linker molecules for selective metallisation of nucleic acids and their uses**

(57) The present invention is related to the linker molecules comprising one or more nucleic acid binding group and one or more nanoparticle binding group which are connected covalently by a spacer group. The problem underlying the present invention is to provide methods for the controlled and selective metallisation of nucleic acids, the production of nanowires which may be used, e. g., in the formation of electronic networks and circuits allowing a high density arrangement, and the components of devices that may be incorporated in such networks and circuits. This problem is solved by a linker molecule which comprises one or more nucleic acid binding group(s) and one or more nanoparticle binding group(s) which are connected covalently by a spacer group. Such linkers can be used for the manufacture of nucleic acid/linker conjugates, nanoparticle/linker conjugates, and nanoparticle/linker/nucleic acid composites and further nanowires, electronic networks, electronic circuits and junctions comprising said nanowires.

EP 1 215 199 A1

**Description**

The present invention is related to the linker molecules comprising one or more nucleic acid binding group and one or more nanoparticle binding group which are connected covalently by a spacer group, the manufacture of nucleic acid/linker conjugates, nanoparticle/linker conjugates, and nanoparticle/linker/nucleic acid composites obtainable therefrom, a method for the manufacture of nanowires, the nanowires obtainable therefrom, electronic networks, electronic circuits and junctions comprising said nanowires.

The electronics industry is constantly progressing towards ever-higher density wiring and circuitry. A key issue in maintaining this progress is to make the individual wires as small as possible. One approach known in the prior art is the metallisation of nucleic acids which, once metallised, serve as an electrically conducting wire.

Strategies for designing molecules capable of targeting DNA, as well as specific regions or segments within the DNA molecules, are well known. A number of anticancer drugs in current medical use owe their effectiveness to their ability to bind to DNA. The binding modes of these drugs can be generally classified as either covalent (irreversible) or non-covalent (reversible). The non-covalent binding modes are also subdivided broadly into three classes: (i) intercalation between adjacent base pairs, (ii) external binding in a groove (usually in the minor groove of AT-rich regions), and (iii) external binding by purely electrostatic interactions with the phosphate backbone. Molecules that bind to DNA from natural sources also tend to bind to synthetic DNA, RNA, oligonucleotides, etc., although the binding mode may vary.

For example, the "metalation" of nucleic acids is known, which refers to the process of direct bonding between a metal atom and a site within the nucleic acid, especially to the N-7 atoms of the purine nucleotides (G and A). Such reactions have been widely studied because of their relevance to the mechanisms of anti-cancer drugs, mostly Pt (II) or Pt (IV) complexes ("platination"). Other metal complexes exhibiting this behavior include the complexes of Pd, Ru, Au, and Rh. The complex requires at least one "labile" ligand as a "leaving group" in order to bind in this manner.

WO 99/04440, published on January 28, 1999, describes a three-step process for the metallisation of DNA. First, silver ions ($Ag^+$) are localized along the DNA through $Ag^+/Na^+$ ion-exchange and formation of complexes between the $Ag^+$ and the DNA nucleotide bases. The silver ion/DNA complex is then reduced using a basic hydroquinone solution to form silver nanoparticles bound to the DNA skeleton. The silver nanoparticles are subsequently "developed" using an acidic solution of hydroquinone and $Ag^+$ under low light conditions, similar to the standard photographic procedure. This process produces silver wires with a width of about 100 nm with differential resistance of about 10 M$\Omega$.

However, a width of 100 nm and particularly a differential resistance of about 10 M$\Omega$ of silver wires produced according to the process described in WO 99/04440 does not meet the need of industry in relation to high density wiring and high density circuits.

The metallisation procedure described in WO 99/04440 is similar to procedures for detecting fragments of DNA by silver staining. Such procedures are known to result in non-specific staining of the DNA fragments and do not distinguish between different DNA sequences. The ability to metallise certain regions on nucleic acid strands and not others may be critical for the development of DNA-based nanoelectronic devices.

Further, Pompe et al. (Pompe et al. (1999) Z. Metallkd. **90,** 1085; Richter et al. (2000) Adv. Mater. **12,** 507) describe DNA as a template for metallisation in order to produce metallic nanowires. Their metallisation method involves treating DNA with an aqueous solution of $Pd(CH_3COO)_2$ for 2 hours, then adding a solution of dimethylamine borane (DMAB) as reducing agent. Palladium nanoparticles with a diameter of 3-5 nm grow on the DNA within a few seconds of the reducing agent being added. After about 1 minute, quasi-continuous coverage is achieved, with metallic aggregates being 20 nm in size. Although this procedure represents a significant advance over the one in WO 99/04440, the initially grown palladium nanoparticles are nonetheless substantially wider than DNA itself (approx. 2 nm for double-stranded DNA).

Accordingly, the problem underlying the present invention is to provide methods for the controlled and selective metallisation of nucleic acids, the production of nanowires which may be used, e. g., in the formation of electronic networks and circuits allowing a high density arrangement, and the components of devices that may be incorporated in such networks and circuits.

This problem is solved by a linker molecule which comprises one or more nucleic acid binding group(s) and one or more nanoparticle binding group(s) which are connected covalently by a spacer group.

In a preferred embodiment, said nucleic acid binding group is selected from the group of agents comprising intercalating agents, groove-binding agents, alkylating agents, and combinations thereof.

In a further preferred embodiment of a linker molecule according to the invention, the intercalating agent is selected from the group of compounds comprising acridines, anthraquinones, diazapyrenium derivatives, furanocoumarins (psoralens), naphthalene diimides, naphthalene monoimides, phenanthridines, porphyrins, and metal coordination complexes containing planar, aromatic ligands (metallointercalators).

Further preferred is a linker molecule, wherein the groove-binding agent is selected from the group of compounds comprising bis-benzamidines, bis-benzimidazoles, lexitropsins, perylene diimides, phenylbenzimidazoles, porphyrins, pyrrole oligopeptides, and viologens.

Further preferred is a linker molecule, wherein the alkylating agent is selected from the group of compounds comprising aziridines, 2-chloroethane derivatives, epoxides, nitrogen mustards, sulfur mustards and metal coordination complexes having at least one leaving group ligand.

Further preferred are linker molecules according to the invention, wherein the metal coordination complexes that are alkylating agents are selected form the group of complexes comprising $Pt^{2+}$, $Pt^{4+}$, $Pd^{2+}$, $Au^+$, $Au^{3+}$ $Ru^{2+}$, $Ru^{3+}$, $Rh^+$, $Rh^{2+}$, and $Rh^{3+}$ having at least one ligand selected from the group comprising halide, (dialkyl)sulfoxides, dicarboxylates, β-diketonates, carboxylates, alkenes, selenate, squarate, ascorbate, and hydroxide.

In the linker molecules according to the invention, the nanoparticle binding group may form covalent bonds with surface ligands on the nanoparticle or displace existing surface ligands on the nanoparticle, or combinations of both.

In a still further embodiment the nanoparticle binding group comprises at least one covalent bond-forming functional group selected from carboxylic acids and derivatives thereof, sulfonic acids and derivatives thereof, amines, alcohols, thiols, aldehydes, ketones, isocyanates, isothiocyanates, ethers, and halides.

Further preferred are linker molecules, wherein the nanoparticle binding group comprises at least one metal-binding group selected from amines, phosphines, thiols, disulfides, dithiocarbamates, dithiophosphates, dithiophosphonates, thioethers, thiosulfates, and thioureas.

In another aspect of the invention, the problem of the invention is solved by a method for the manufacture of a nanoparticle/linker conjugate, wherein a nanoparticle is combined with a linker molecule according to the invention, thereby forming a nanoparticle/linker conjugate. The invention further includes a method for the manufacture of a nanoparticle-nucleic acid composite, wherein the nanoparticle/linker conjugate is further reacted with a nucleic acid, forming a nanoparticle-nucleic acid composite.

In still another aspect of the invention, the problem of the invention is solved by a method for the manufacture of a nucleic acid/linker conjugate, wherein a nucleic acid molecule is reacted with a linker molecule according to the invention, thereby forming a nucleic acid/linker conjugate. Also in this aspect, the invention further includes a method for the manufacture of a nanoparticle-nucleic acid composite, wherein the nucleic acid/linker conjugate is further reacted with a nanoparticle, forming a nanoparticle-nucleic acid composite.

According to one embodiment of the invention, the above-mentioned methods can further be characterized in that the nucleic acid is present dissolved in solution, preferably in an aqueous solution, or immobilized on a substrate, preferably a non-metallic substrate or an electrode structure.

A further preferred embodiment of a method according to the invention is characterized in that the nucleic acid is selected from the group comprising natural, modified, synthetic, and recombinant nucleic acids, like oligonucleotides, primers, DNA, RNA, homoduplexes, heteroduplexes, triplexes, quadruplexes or multiplexes, T-junctions of nucleic acids, domains of non-nucleic acid polymer-nucleic acid blockcopolymers and combinations thereof. Suitable non-nucleic acid polymers for blockcopolymers can be polypeptides, polysaccharides, like dextran or artificial polymers, like polyethyleneglycol (PEG) and are generally known to the person skilled in the art. Preferably, the nucleic acid is double-stranded or single-stranded.

In a preferred embodiment of a method according to the invention, the nanoparticle is catalytically active towards electroless plating

Another embodiment of the method according to the invention is characterized in that the nanoparticle contains a metal selected from the group comprising Fe, Co, Ni, Cu, Ru, Rh, Pd, Os, Ir, Pt, Ag, Au and alloys thereof.

In further preferred embodiments of the inventive methods the nanoparticle's size is less than 10 nm. Most preferred is a method in which the nanoparticle's size is between about 0.5 nm and about 3 nm.

Another aspect of the present invention is related to a nanoparticle comprising conjugate or nucleic acid comprising conjugate obtainable according to one of the inventive methods. Further, a nanoparticle-nucleic acid composite obtainable according to one of the inventive methods is included in the scope of the invention.

Still another aspect of the invention is a method for the manufacture of a nanowire, comprising electroless deposition of a metal onto a nanoparticle-nucleic acid composite according to the invention.

Another embodiment of this inventive method is characterized in that the nanoparticle-nucleic acid composite is present dissolved in solution, preferably in an aqueous solution or immobilized on a substrate, preferably a non-metallic substrate or an electrode structure.

The metal deposited by electroless plating can be selected from the group comprising Fe, Co, Ni, Cu, Ru, Rh, Pd, Os, Ir, Pt, Ag, Au and alloys thereof.

Further, the metal deposited by electroless plating can be selected from the group comprising magnetic and/or magnetized Fe, Co, Ni and alloys thereof, and alloys thereof with B or P, in order to obtain magnetic nanostructures.

In a still further aspect the problem is solved by a nanowire which is obtainable by one of the inventive methods. The inventive nanowires can form an electronic network comprising at least one nanowire or an electronic circuit comprising at least one electronic network according to the invention.

In addition, the inventive nanowires can be used as electronic components in their not completely metallised form, in which more or less insulating spaces are present between the individual nanoparticles positioned along the nucleic acid strand. In another aspect the nanowires may be fully conducting or may contain insulating parts either at one or both ends, or the insulating parts may be within the wire itself, so that the nanowire is comprised of single conducting islands. These inventive structures can form or can be part of an electronic network or an electronic circuit comprising at least one nanowire. In such electronic networks or electronic circuits, junctions between two or more wires may be formed, wherein each of the wires has a connecting segment proximal to the junction comprising the nanowire. Further, the nanowire comprising networks may be parts of hybrid electronic structures.

In a still further aspect the problem is solved by the use of the inventive method for selective metallisation of a nucleic acid.

The present invention relates to a new method for the manufacture of nucleic acid-templates nanowires. Specifically, the invention provides new compounds (referred to singly herein as "linker molecule" or "linker") that surprisingly allow for the coupling of metal-based nanoparticles to nucleic acids using principles of molecular self-assembly. Nanoparticles are also referred to in the prior art as "clusters", "nanoclusters", or "nanocrystals", depending to some extend on their size and crystallinity. All such species are referred to herein simply as "nanoparticles".

The nanoparticles employed in the present invention are catalytically active towards electroless deposition of metal, also known as "electroless plating". As demonstrated in the examples below, coupling of nanoparticles and nucleic acids by means of linker molecules according to the present invention provides nanoparticle-nucleic acid composites in which nanoparticles are aligned along the nucleic acid backbone. Although such chains of nanoparticles can serve as nanowires, a preferred embodiment of the invention is to treat the nanoparticle-nucleic acid composite with an electroless plating bath solution in order to enlarge the particles, thereby enhancing the conductivity or imparting them with magnetic properties. The manufacture of this kind of electroless plating treated nucleic acid/linker/nanoparticle composites then allows the manufacture of nanowires which can advantageously be used for the construction of high density networks and circuits. The benefits arise mostly from the reduced diameter of such nanowires compared to the ones obtainable according to the methods of the prior art.

Further, the inventors of the present application have surprisingly found that by using such a nucleic acid/linker/nanoparticle composite or electroless plating treated nucleic acid/linker/nanoparticle composites a process for selective metallisation of nucleic acids and oligonucleotides is provided which, due to the selectivity, allows a controlled metallisation of metallised nucleic acids providing an electrically conductive path, i. e. a wire, more particularly a nanowire.

The invention is now further illustrated by the accompanying figures from which further embodiments, features and advantages may be taken.

Figure 1    shows a schematic representation of linker molecule according to the present invention;

Figure 2    illustrates the structures of the four bases in their form as base pairs of DNA, also illustrating specific hydrogen bonding interactions (the thymine group is replaced by uridine in RNA);

Figure 3    shows a schematic representation of the assembly of nucleic acid, linker molecule, and nanoparticle into a nanoparticle-nucleic acid composite according to the present invention. The intermediate combinations, referred to as linker/nucleic acid and linker/nanoparticle conjugates, are also shown;

Figure 4    shows the structures of specific linker molecules based on an intercalating agent (anthraquinone), a groove-binding agent (cationic porphyrin), and an alkylating agent (nitrogen mustard);

Figure 5    shows the structures of specific linker molecules based on platinum and palladium coordination complexes as alkylating agents;

Figure 6    shows two AFM images of a nanoparticle-nucleic acid composite of the invention;

Figure 7    shows two AFM images from a control experiment;

Figure 8    shows an AFM image of a nanoparticle-nucleic acid composite of the invention after electroless plating;

Figure 9    shows two AFM images of a nanoparticle-nucleic acid composite of the invention before (a) and after (b) electroless plating;

Figure 10    also shows two AFM images of a nanoparticle-nucleic acid composite of the invention before (a) and after (b) electroless plating;

Figure 11    shows two AFM images from a control experiment; and

Figure 12    shows AFM images of (a) a linker/nucleic acid conjugate of the invention and (b) a nanoparticle/nucleic acid composite of the invention.

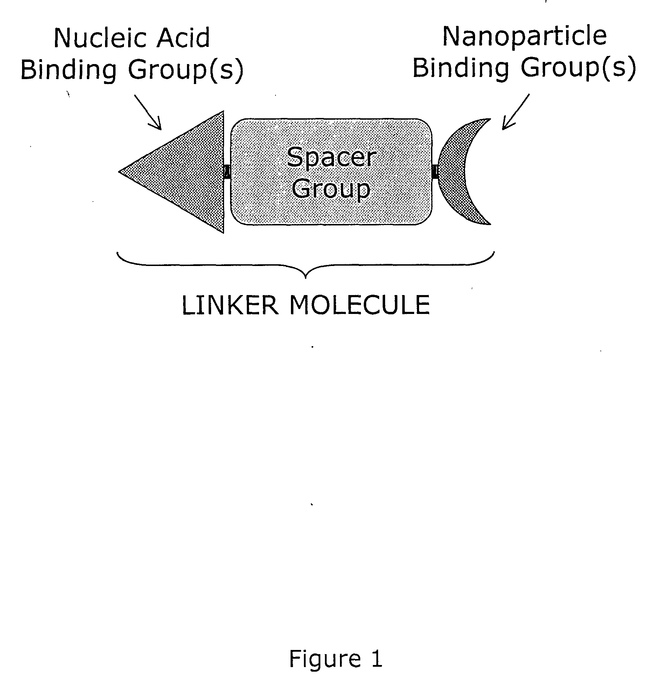

The general features of the linker molecules provided by the present invention are indicated in Figure 1. Essentially, the linker possesses one or more nucleic acid binding group and one or more nanoparticle binding group. As indicated, the two classes of binding groups are segregated within the linker molecule, separated by a spacer group. The main function of the spacer group is to provide the molecular framework necessary for connecting the nucleic acid binding group(s) to the nanoparticle binding group(s). From prior art, the inventors anticipate that, for any particular set of binding groups, there will be an optimal distance and orientation between them. Prior art also suggests that including within the spacer group certain sub-groups such as amines, which can interact with the sugar-phosphate backbone of the nucleic acid, can be advantageous. The optimal molecular characteristics of the spacer group can not be predicted a priori, however, since they would depend on the nature of the nucleic acid binding group(s) and nanoparticle particle binding group(s) and the means of connecting them. The main function of the spacer group, that of a connector, can therefore be fulfilled by a variety of groups, including single bonds, alkyl chains, aromatic rings, peptides, etc. In general, the distance between the two binding regions of the linker molecule is preferred to be greater than 0.3 nm and less than 3 nm.

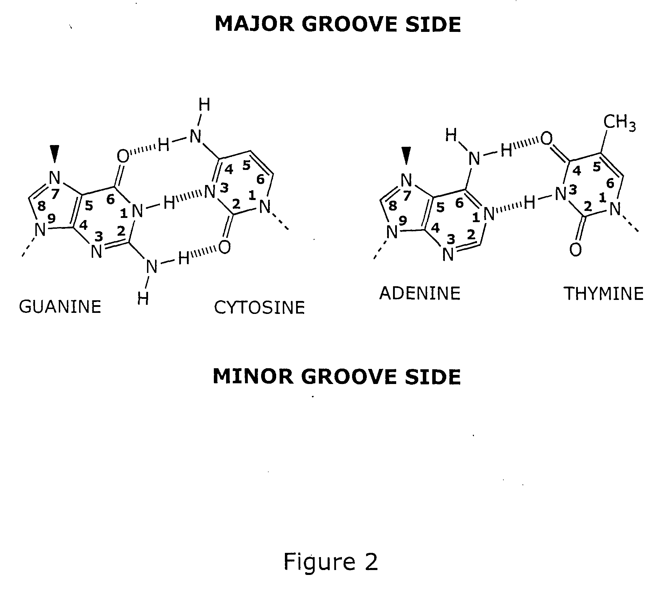

The nucleic acid binding group(s) attached to the linker molecules provided by the present invention fall into three classes: intercalating agents, groove binding agents, and alkylating agents. From the prior art, it is known that each type of binding agent provides a particular binding affinity and selectivity towards nucleic acids. Intercalating agents generally tend to bind to segments of nucleic acids that are double-stranded and GC-rich. Groove binding agents generally tend to bind to segments of nucleic acids that are double-stranded and AT-rich. The structures of the guanine:cytosine (GC) and the adenine:thymine (AT) base pairs are shown in Figure 2. Alkylating agents bind covalently to nucleic acids, tending to form covalent adducts at the N-7 atoms of G and A irrespective of whether the nucleic acid is double-stranded or single-stranded. The N-7 positions are indicated by black triangles in Figure 2.

It is further known from the prior art that the binding affinity and selectivity of molecules towards nucleic acids can be tuned by combining within the same molecule two or more binding agents. These can be of the same class or different classes. Thus, by careful selection of the nucleic acid binding group(s), the linker molecule provided by the present invention can be tuned to target specific nucleic acids and domains thereof. This targeting ability makes it possible to selectively metallise nucleic acids and manufacture nanowires therefrom, using further embodiments provided by the present invention.

Intercalating agents and external (groove) binding agents interact non-covalently with nucleic acids. Despite being reversible binding processes, the binding constants can be quite high. Widely known intercalating agents in the prior art include acridines, anthraquinones, and phenanthridines. A preferred embodiment of the present invention is to use linker molecules that are derivatives of 9,10-anthraquinone. It is shown in the prior art, that a variety of intercalating agents can be synthesized in straightforward manner from commercially available substituted anthraquinones. Groove binding agents known from the prior art include bis-benzimidazoles, such as the dyes commercially available from Hoechst, Frankfurt, Germany and used as DNA probes, phenylbenzimidazoles as disclosed in US 5,821,258 and pyrrole oligopeptides, such as the naturally occurring antibiotics netropsin and distamycin. More recently, much attention has been directed towards cationic porphyrins as nucleic acid binding agents, because the binding mode can easily be tuned to be the intercalative or external type by varying the metal center and peripheral substituents. A preferred embodiment of the present invention is to use linker molecules containing cationic porphyrin groups that bind externally to nucleic acids.

The present invention utilizes two general classes of alkylating groups as nucleic acid binding groups in the linker molecules: organic compounds and coordination complexes of metal ions. These two classes of compounds provide considerable flexibility with respect to the linker molecules that can be synthesized. Widely known organic alkylating agents in the prior art are aziridines, epoxides, and 2-chloroethane derivatives. Examples of the latter are the so-called nitrogen mustards and sulfur mustards. A preferred embodiment of the present invention is to use linker molecules that are derivatives of the nitrogen mustard drug known as "chlorambucil" (4-[bis(2-chloroethyl)amino]benzenebutanoic acid). It is shown in the prior art that the carboxylic acid group of this compound is readily coupled to the alcohol or amine groups of other compounds. Coordination complexes of platinum(2+) are perhaps the best known DNA alkylating agents because of their widespread use as anticancer drugs, and the best known of these is the one that was discovered first, "cisplatin" (cis-diamminedichloroplatinum(II)). These complexes contain at least one ligand that serves as "leaving group", making it possible for the platinum ion to bind to e.g., the N-7-positions in guanine and adenine. Another preferred embodiment of the present invention is to use linker molecules that are platinum complexes related to cisplatin. Several other metals, notably palladium, gold, ruthenium and rhodium, are also known to form coordination complexes with nucleic acid alkylating properties and can be used instead of platinum. Examples for the synthesis of mixed-ligand platinum(II) complexes are disclosed, for example, in US 5,985,566 and US 6,110,907. Another preferred embodiment of the present invention is to use linker molecules that are platinum or palladium complexes having at least one leaving group and at least one non-coordinated ("dangling") nanoparticle binding group.

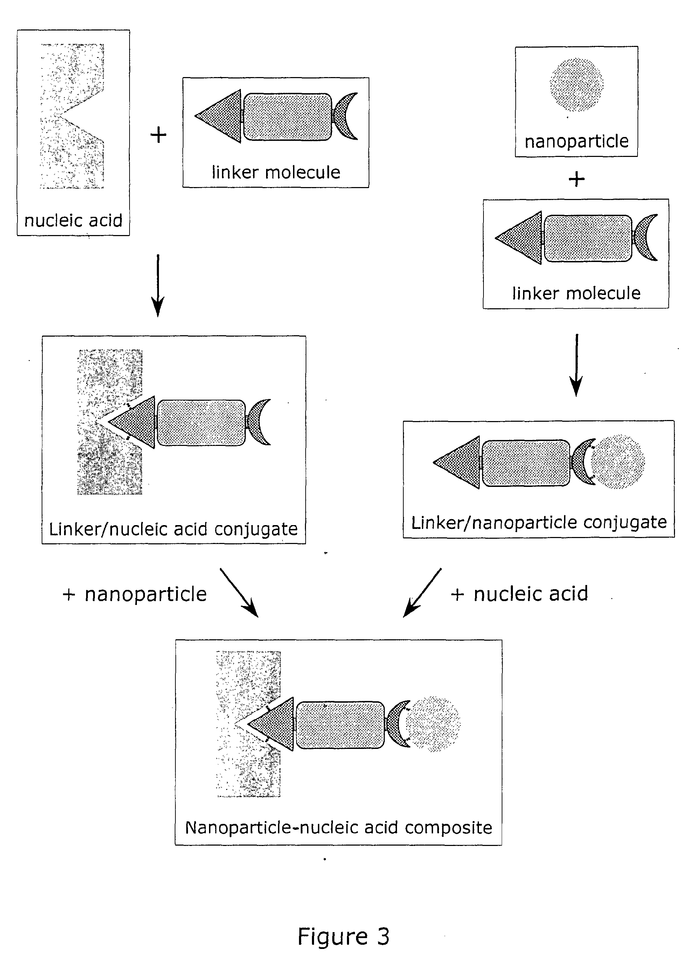

A general outline of the process for the manufacture of a nanoparticle-nucleic acid composite is shown in Figure 3. In general, two different routes for the production of the composite can be chosen which lead to different intermediate conjugates. In a first embodiment, a nanoparticle is combined with a linker molecule according to the invention, thereby forming a nanoparticle/linker conjugate (Figure 3, right pathway). In a second embodiment, a nucleic acid molecule is reacted with a linker molecule according to the invention, thereby forming a nucleic acid/linker conjugate (Figure 3, left pathway). Both conjugates can then be combined with either the missing nucleic acid or nanoparticle in order to obtain the desired nanoparticle/linker/nucleic acid composite. The nanoparticle/linker/nucleic acid composite can then be present dissolved in solution, preferably in an aqueous solution or immobilized on a substrate, preferably a non-metallic substrate or an electrode structure.

The nucleic acid used in the nucleic acid conjugates or composite, herein referred to also as nucleic acid molecule, may be either DNA or RNA. This interchangeability, which applies for many cases, resides in physicochemical similarities between DNA and RNA. Of course, any polymers or derivatives thereof can also be used in the present invention such as, but not limited to, oligonucleotides of DNA and RNA, respectively, primers thereof and polynucleotides of each of said two nucleic acid species. Additionally, nucleic acids which can be used in the present invention may show various conformations such as A-DNA, B-DNA and Z-DNA which differ mostly in the diameter and the particular kind of helix structure, T-junctions of nucleic acids, domains of non-nucleic acid polymer-nucleic acid blockcopolymer and combinations thereof. Suited non-nucleic acid polymers for blockcopolymers can be polypeptides, polysaccharides, like dextran or artificial polymers, like polyethyleneglycol (PEG) and are generally known to the person skilled in the art. It is to be understood that any of the aforementioned nucleic acid species may be in single-, double-, or multi-stranded form. In connection with this it is particularly referred to the various configuration of nucleic acids such as A-DNA, B-DNA and Z-DNA or other structures, such as hammerhead structures and the like, which arise mostly from single-stranded nucleic acid molecules.

The nanoparticle as such has a composition that is catalytic towards electroless metallisation so that it enables enlargement in the presence of a suitable combination of metal ion and reducing agent. Also, suitable nanoparticles are stable towards aqueous solutions under ambient conditions. Based on these constraints, the nanoparticle preferably consists of one of the metals Fe, Co, Ni, Cu, Ru, Rh, Pd, Os, Ir, Pt, Ag or Au and alloys thereof. The metal deposited by electroless plating can be selected from the group comprising Co, Ni, Cu, Ru, Rh, Pd, Pt, Ag, Au and alloys thereof. Further, it is possible to also use magnetic and/or magnetized metal particles, like Fe, Co, Ni or alloys thereof, or alloys thereof with B or P.

The nanoparticle may also consist of precursor(s) of these metals which is (are) reduced to the catalytic (metallic) state by the reducing agent in the electroless metallisation bath. Actually, in most of the embodiments of the invention any nanoparticle can be used which meets the following criteria: (i) a diameter of about 10 nm or less, preferably between about 3 nm and 0.5 nm (ii) stability and solubility in water (or suitable aqueous-organic solvent mixtures or solvents compatible with nucleic acids), (iii) capability of surface modification, and (iv) capability of enlargement via electroless deposition. The particles are small enough that they may be referred to as "clusters", and it is not necessary that the metal atoms constituting the particles or clusters be entirely in the zerovalent state. A great deal of literature exists concerning the synthesis and properties of metal-based nanoparticles and clusters. Some of biologically related applications are discussed in EP 0 426 300 the disclosure of which is incorporated herein by reference.

Nanoparticle/cluster materials are also commercially available by Nanoprobes, Inc (Stony Brook, New York), see also US 5,728,590 the disclosure of which is incorporated herein by reference. Also available are water-soluble gold nanoparticles with amino and/or carboxylic acid groups on the surface which provide for a covalent linkage between the linker molecule and the nanoparticle.

The inventive linker/nucleic acid conjugates and linker/nanoparticle conjugates and nanoparticle/nucleic acid composites derived therefrom, as well as the inventive methods for producing the same form the basis for the selective metallisation of nucleic acids. By any of the means proposed herein, selectivity may be achieved by providing appropriate nucleotide sequences targeted by the linker molecules. Metallisation methods according to the state of the art lack such specificity to metallise certain regions of nucleic acids.

In the manufacture of a nanowire providing a nucleic acid with nanoparticle being catalytic towards electroless metallisation is a key step insofar as the distribution of such nanoparticles along the nucleic acid strand is critical for the metallisation process. The metallisation process being considered in this invention is an "electroless" (non-electrical) one. Electroless metal deposition ("metallisation" or "plating") is a process in which a metal is deposited on a substrate surface from a solution without using an external source of electric current. The process is a redox reaction in which metal ions (or complexes) are chemically reduced to the metal on the surface. Although the reaction between the metal ion and reducing agent is thermodynamically spontaneous, it is initiated selectively at surface sites "seeded" with a catalyst. Subsequently each layer of freshly deposited metal becomes the catalyst for deposition of the next layer ("autocatalysis"). The catalyst used most often in the electroless copper plating on printed circuit boards is Pd metal, but other metals such as Pt and Au can also be used.

A reducing agent often used in the electroless copper plating on printed circuit boards is formaldehyde, the use of which is illustrated in reaction 1.

$$Cu^{2+} + 2H_2CO + 40H^- \rightarrow Cu^0 + H_2 + 2HCOO^- + 2H_2O \qquad (1)$$

In these solutions the copper ions are complexed with an organic ligand in order to increase their solubility in the highly alkaline solution. Other reducing agents such as hypophosphite and dimethylaminoborane are also used. Examples of electroless plating processes are further disclosed in, for example, US 5,318,621; US 5,292,361 and US 5,306,334.

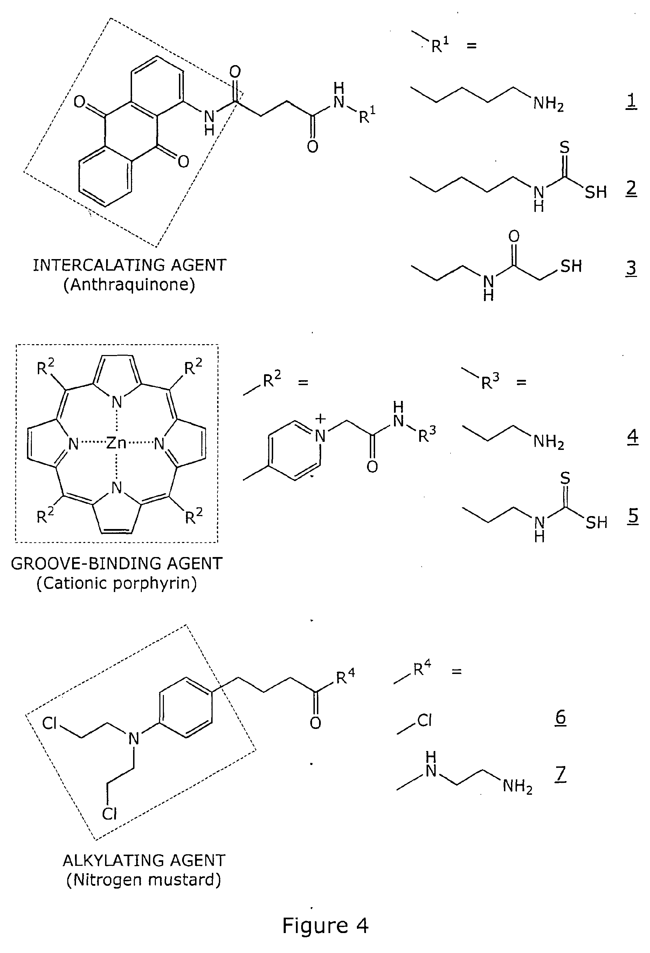

**Figure 4** indicates the structures of seven compounds suitable as linker molecules according to the present invention. These compounds contain as nucleic acid binding groups at one end either anthraquinone (an intercalating agent), a cationic porphyrin (a groove-binding agent) or a nitrogen mustard (an alkylating agent). The nanoparticle binding groups at the other end of the linker molecules consist of either amine (-NH$_2$), dithiocarbamate (-NHC(S)SH), thiol (-SH) or acid chloride (-COCl). The amine groups in compounds 1, 4 and 7 can bind directly to the surface of gold or platinum nanoparticles, for example; they can also react covalently with the activated carboxylic acid groups of *sulfo*-N-hydroxy-succinimido Nanogold® particles, for example. The thiocarbamate groups of compounds 2 and 5 and thiol group of compound 3 can bind directly to the surface of gold, silver, or platinum nanoparticles, for example. The thiol group of compound 3 can also react covalently with the maleimido group of monomaleimido Nanogold® particles, for example. The acid chloride group of compound 6 is an activated species that can react covalently with the amine group of monoamino Nanogold®, for example.

Compound 1 is synthesized by reacting 1-amino-9,10-anthraquinone (AQ-NH$_2$) with excess succinyl dichloride (ClOCCH$_2$CH$_2$COCl) to obtain AQ-NHCOCH$_2$CH$_2$COCl, which is then reacted with excess 1,4-diaminobutane (H$_2$NCH$_2$CH$_2$CH$_2$CH$_2$NH$_2$). The excess reagents are removed after each step to prevent the formation of by-products. Preferred solvents are dichloromethane (CH$_2$Cl$_2$) and N,N-dimethylformamide (CHCON(CH$_3$)$_2$); the preferred reaction temperature is 20-80 °C and time is 1-4 hours. The ammonium salt of compound 2 is prepared by reacting 1 with carbon disulfide (CS$_2$) and ammonia (NH$_4$OH). The preferred solvent is ethanol (CH$_3$CH$_2$OH); the preferred reaction temperature is 20-40 °C and time is 1-2 hours. Compound 3 is synthesized by combining the intermediate AQ-NHCOCH$_2$CH$_2$COCl with excess 1,2-diaminoethane (H$_2$NCH$_2$CH$_2$NH$_2$) to obtain AQ-NHCOCH$_2$CH$_2$-CONHCH$_2$CH$_2$NH$_2$. The excess reagents are removed after each step to prevent the formation of by-products. The compound AQ-NHCOCH$_2$CH$_2$CONHCH$_2$CH$_2$NH$_2$ is converted to compound 3 by reacting it sequentially with bromoacetylbromide (BrCH$_2$COBr), potassium thioacetate (KSCOCH$_3$), sodium methylate (NaSCH$_3$), and hydrochloric acid (HCl). The preferred solvents are N,N-dimethylformamide and methanol (CH$_3$OH); the preferred reaction temperature is 20-80 °C and time is 0.5-2 hours.

Compound 4 is synthesized by reacting zinc(II)-5,10,15,20-tetrakis-(4-pyridyl)-21H,23H-porphine (ZnP(Py)$_4$) with excess bromoacetylbromide to obtain [ZnP(Py-CH$_2$COBr)$_4$]Br$_4$. The preferred solvent is N,N-dimethylformamide; the preferred reaction temperature is 150 °C (reflux) and time is 3-10 hours. The product is isolated from excess bromoacetylbromide and by-products by precipitation with acetone (CH$_3$COCH$_3$) and it is reacted immediately thereafter with excess 1,2-diaminoethane to form compound 4. The preferred solvent is N,N-dimethylformamide; the preferred reaction temperature is 10-30 °C and time is 0.5-1 hour. The product is isolated from excess 1,2-diaminoethane and hydrogen bromide (HBr) salts by precipitation with acetone. The ammonium salt of compound 5 is prepared by reacting 4 with carbon disulfide and ammonia. The preferred solvent is ethanol; the preferred reaction temperature is 20-40 °C and time is 1-4 hours.

Compound 6 is prepared from chlorambucil (4-[bis(2-chloroethyl)amino]benzenebutanoic acid) by reacting it with excess oxalyl chloride (ClOCCOCl). The preferred solvent is dichloromethane; the preferred reaction temperature is 30-40 °C and time is 10-20 hours. The compound is isolated by evaporation to dryness and is used immediately thereafter. Compound 7 is synthesized from compound 6 by reacting it with excess 1,2-diaminoethane. The preferred solvent is dichloromethane; the preferred reaction temperature is 0-20 °C and time is 0.5-1 hour. The product is isolated from excess 1,2-diaminoethane and hydrogen bromide (HBr) salts by evaporating the reaction mixture to dryness and washing the solid with water.

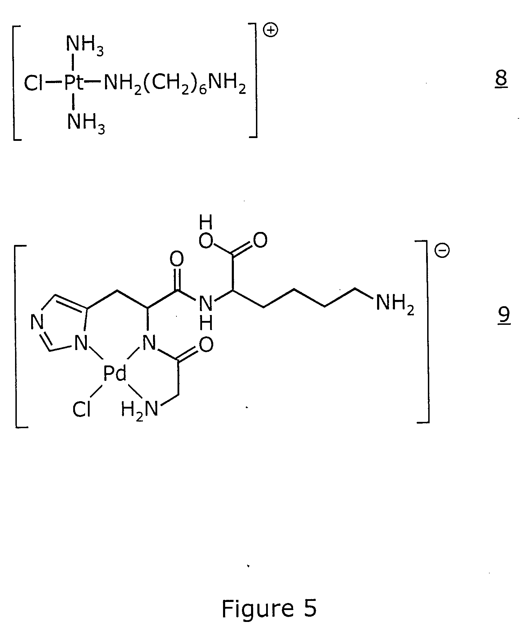

**Figure 5** indicates the structures of two metal coordination compounds (8 and 9) suitable as linker molecules according to the present invention. These compounds contain as nucleic acid binding groups at one end Pt(II) or Pd (II) complexes with a chloride ion ligand as leaving group. The nanoparticle binding group at the other end of these linker molecules is the amino ($-NH_2$) group, which can react covalently with the activated carboxylic acid groups of *sulfo*-N-hydroxy-succinimido Nanogold® particles, for example. The synthesis of compound 8 is described by Valsecchi et al. in US 5,744,497 and the synthesis of compound 9 is described by Rombeck and Lippert in Inorganica Chimica Acta **273,** 31 (1998).

The specific compounds (1-9) in Figures 4 and 5 are presented solely as representative examples conforming to the general class of linker molecules indicated in Figure 1.

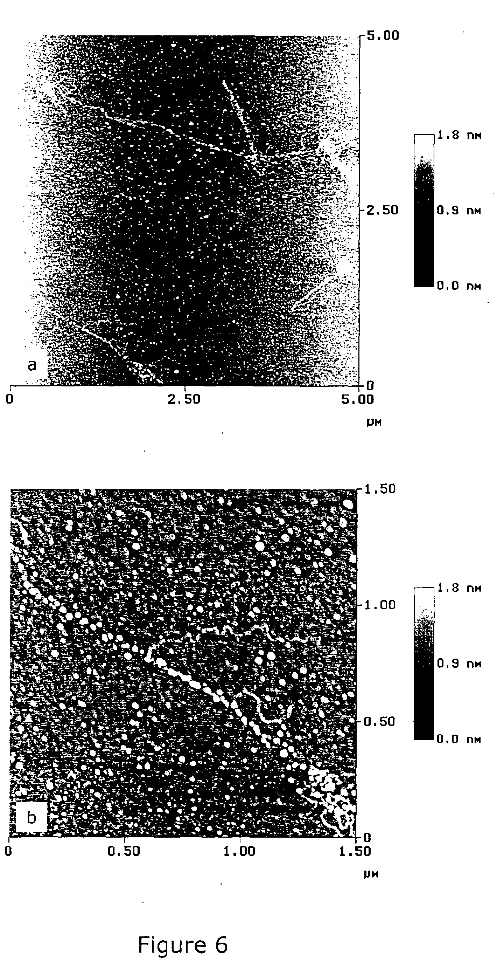

**Figures 6-12** are AFM images illustrating the formation of nanoparticle/nucleic acid composites using intercalating linker molecules (1 or 3) and subsequent growth of the nanoparticles by electroless plating. Also included are results from control experiments. In these experiments, calf thymus DNA is used as the nucleic acid component and GoldEnhance® is used as the electroless plating bath solution. The nanoparticle component is either Nanogold® or phosphine-stabilised gold nanoparticles, which were prepared according to the procedure described by Duff et al. in Langmuir **9,** 2302 (1993). Experimental details are given in **Examples 1-10.**

**Figure 6** shows AFM images of the nanoparticle/nucleic acid composite obtained via the conjugate between linker molecule 1 and Nanogold® as described in **Example 1** and **Example 2.** The figure shows images scanned over (a) 5 μm x 5 μm and (b) 1.5 μm x 1.5 μm regions. Clear evidence of alignment of particles (approximately 1-1.5 nm in size) along DNA molecules is seen, as well as particles scattered randomly on the substrate.

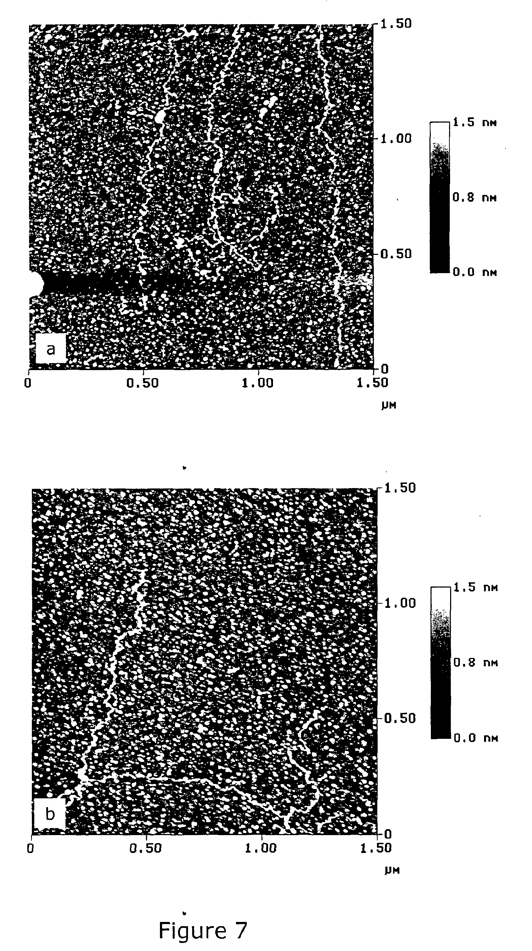

**Figure 7** shows AFM images for a control sample using Nanogold particles without linker, as described in **Example 3;** the two images (a) and (b) were scanned over 1.5 μm x 1.5 μm regions. The density of particles on the substrate is much higher in this case, and there is no clear tendency of the particles to align along the DNA molecules.

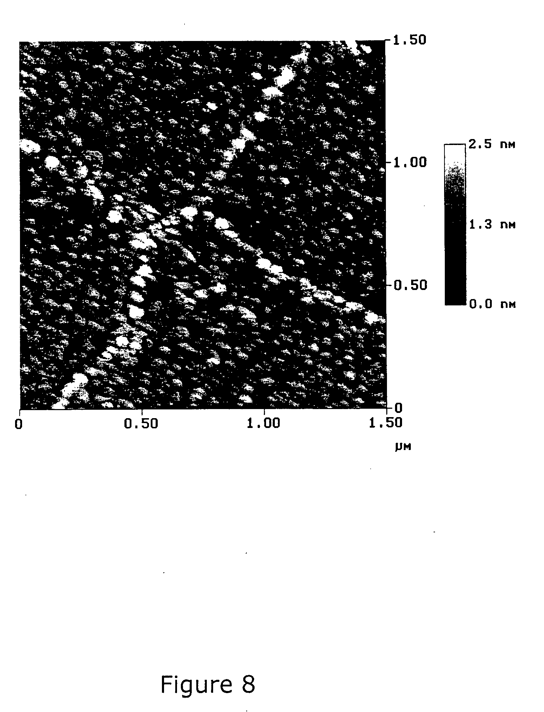

**Figure 8** shows an AFM image obtained after treatment of the sample in Figure 6 with GoldEnhance® solution for 5 minutes, as described in **Example 4**. The image shows that the particles along the DNA had become enlarged by approximately 40%.

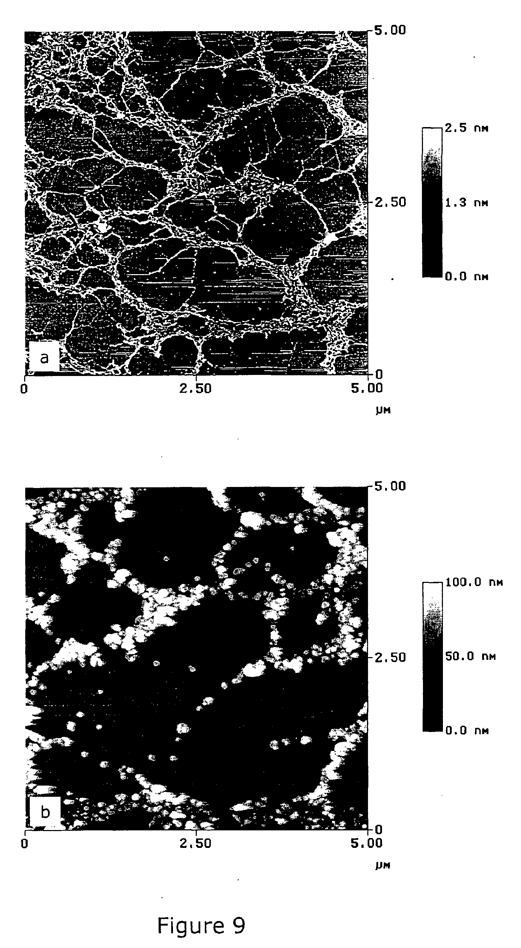

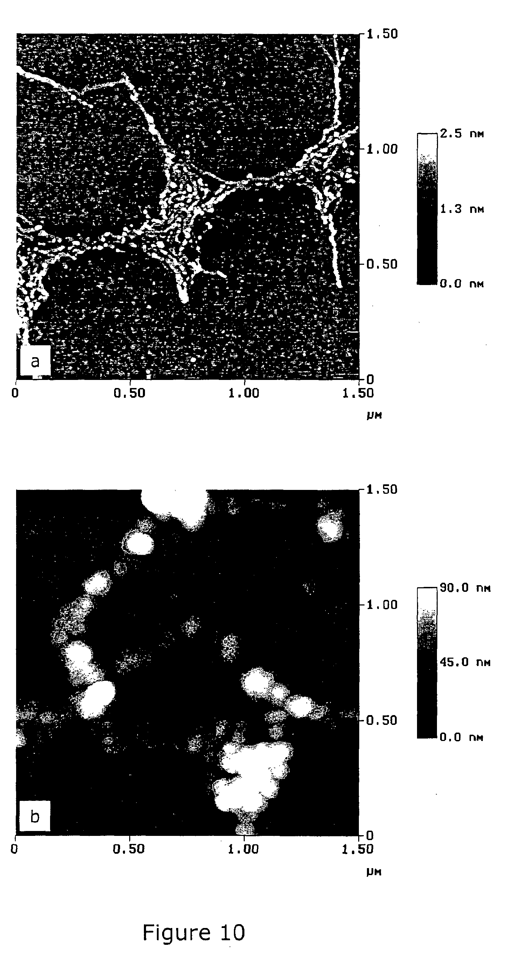

**Figure 9** and **Figure 10** show AFM images of the nanoparticle/nucleic acid composite obtained via the conjugate between linker molecule 3 and calf thymus DNA, before (a) and after (b) treatment with GoldEnhance® solution for 15 minutes. Binding of the phosphine-stabilised gold nanoparticles to the conjugate to form the composite in this experiment was performed in the solution phase. Experimental details are provided in **Examples 5-7.** Figure 9 shows images scanned over a 5 μm x 5 μm region and Figure 10 shows images scanned over a 1.5 μm x 1.5 μm region. The images obtained before electroless plating (a) show interconnected strands of DNA lined with particles (approximately 1-1.5 nm in size). It is apparent that the phosphine-stablized particles have little affinity for the silicon substrate. The images obtained after electroless plating (b) show that the particles grew relatively uniformly to approximately 20-30 nm and there was little, if any, gold deposition on the substrate itself.

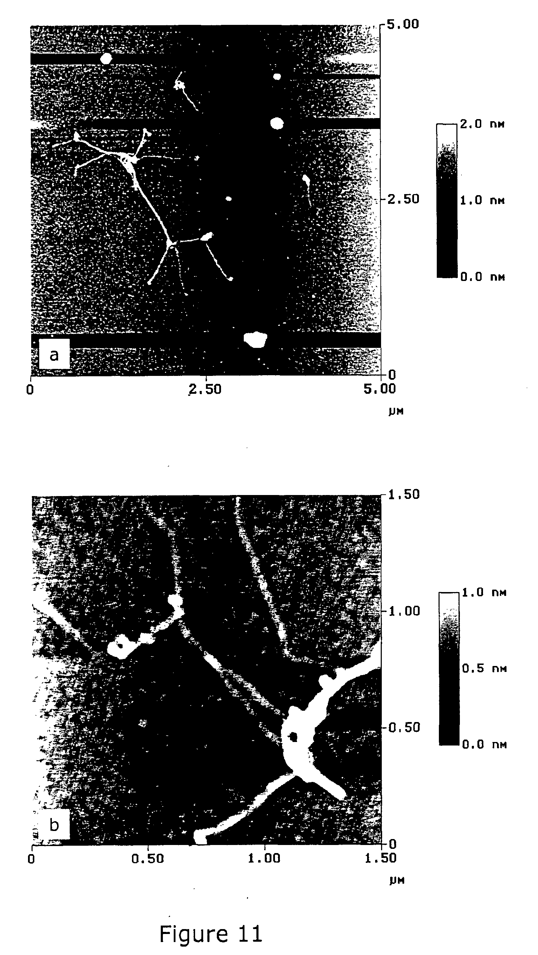

**Figure 11** shows AFM images for a control sample in which calf thymus DNA alone was treated with GoldEnhance® solution for 15 minutes, as described in **Example 8.** There is no clear evidence for particle growth on the DNA in this case.

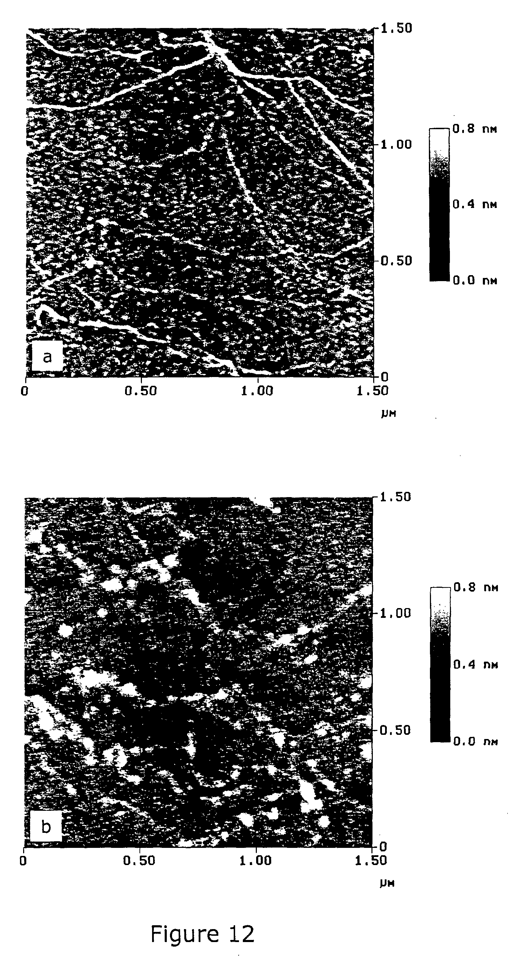

**Figure 12** shows AFM images of the nanoparticle/nucleic acid composite obtained via the conjugate between linker molecule 3 and calf thymus DNA, before (a) and after (b) treatment with a solution of the phosphine-stabilised gold nanoparticles for 1 hour. Experimental details are provided in **Example 9** and **Example 10.** The image in (b) demonstrates that binding of the nanoparticles to form the composite occurs when the linker/nucleic acid conjugate is immobilised on the silicon substrate.

Example 1: Linker/Nanoparticle Conjugate from Compound 1 and Nanogold®

Several milligrams of the anthraquinone derivative 1 were dissolved in approximately 0.5 mL of dimethylsulfoxide. The concentration of 1 in the solution was obtained by UV-visible absorption after dilution, assuming the molar extinction coefficient at 380 nm to be that of an analogous compound reported by Breslin and Schuster in J. American Chemical Soc. **118,** 2311 (1996), 7000 $M^{-1}cm^{-1}$. To a vial containing 6 nmoles of *sulfo*-N-hydroxy-succinimido Nanogold® (Nanoprobes Inc., catalog number 2025A) was added 25 μL of the undiluted solution of 1, containing 30 nmoles of the compound, and 15 μL of dimethylsulfoxide. After mixing to ensure complete dissolution of the Nanogold powder, 160 μL of water was added, and the solution was again mixed, then kept in the dark at room temperature. After 1.3 hours, the solution was chromatographed by gel-filtration (Sephadex G-25, 0.02 M HEPES/NaOH buffer, pH 7.5) to remove the excess, unbound 1 from the gold particles, which eluted first. The gold nanoparticle fraction was diluted with the buffer to a volume of 1.0 mL, and stored at 4 °C.

Example 2: Nanoparticle/Nucleic Acid Composite from the Conjugate in Example 1 and DNA

DNA (from calf thymus, Sigma-Aldrich, product number D-1501) was dissolved in an aqueous solution containing 0.02 M HEPES/NaOH buffer, pH 7.5. The equivalent concentration of nucleotide bases in the solution, estimated by UV-visible absorption, was 80 μM. The polished surface of a piece of silicon wafer (semiconductor grade, p-type, boron doped, with a native surface oxide) was treated with an $O_2$-plasma (Gala Instruments, PlasmaPrep-5) for 4 minutes (0.4 mbar, ca 33 W). The treated wafer was then mounted onto a spin-coater (Mikasa, Spincoater 1H-D3). Several drops of the solution of DNA were applied to cover the substrate. After 2 minutes, the sample was spun at 1000 rpm for 10 seconds, then immediately thereafter at 5000 rpm for 90 seconds. Two drops of water were dropped onto the sample during the second spin stage to remove salts. Several drops of the solution of linker/nanoparticle conjugate from Example 1 were applied to the substrate, and left there for 1 hour before rinsing with water and drying with compressed $N_2$. The sample was examined by tapping-mode AFM (Digital Instruments, MultiMode Atomic Force Microscope) using silicon nitride cantilevers (Olympus Optical, Micro Cantilever OMCL-AC160TS-W, ca 250 kHz resonant frequency, ca 25 N/m spring constant). Examples of the AFM images obtained are shown in **Figure 6.**

Example 3: Control Experiment for Example 2

Nanogold particles without a linker molecule attached were prepared by dissolving *sulfo-N*-hydroxy-succinimido Nanogold® in a dimethylsulfoxide-water mixture, as in Example 1 but without compound 1. This process should simply result in hydrolysis of the *sulfo-N*-hydroxy-succinimido (active ester) moiety on the particle. DNA that had been immobilised on a silicon substrate was treated with the solution of these particles as described in Example 2. Examples of AFM images obtained are shown in **Figure 7**.

Example 4: Electroless Deposition of Gold onto the Nanoparticle-Nucleic Acid Composite from Example 2

GoldEnhance® (Nanoprobes Inc., catalog number 2113) solution was prepared according to the manufacturer's specifications. Several drops of the solution were then applied to the surface of the sample from Example 2, and left there for 5 minutes before rinsing with water and drying with compressed $N_2$. An example of one of the AFM images obtained after this treatment is shown in **Figure 8.**

Example 5: Linker/Nanoparticle Conjugate from Compound 3 and Phosphine-stabilised Gold Nanoparticles

A solution of the anthraquinone derivative 3 was prepared in ethanol; the concentration was 1.3 mM, as estimated by UV-visible absorption after dilution as described in Example 1. 20 µL of this solution was mixed in a small vial with 20 µL of the colloidal solution of phosphine-stabilised gold nanoparticles to obtain a clear solution, which was stored at 4 °C for 18 hours.

Example 6: Nanoparticle-Nucleic Acid Composite from the Conjugate in Example 5 and DNA

DNA (from calf thymus, Sigma-Aldrich, product number D-1501) was dissolved in an aqueous solution containing phosphate buffered saline solution, pH 7.4 (Sigma-Aldrich, product number P-4417). The equivalent concentration of nucleotide bases in the solution, estimated by UV-visible absorption, was 250 µM. 80 µL of this solution was mixed with the solution of linker/nanoparticle conjugate from Example 5 to obtain a clear solution, which was stored at 4 °C for at least 24 hours. This composite was deposited onto plasma-treated silicon for AFM analysis as described in Example 2. AFM images obtained are shown in **Figure 9(a)** and **Figure 10(a).**

Example 7: Electroless Deposition of Gold onto the Nanoparticle-Nucleic Acid Composite from Example 6

GoldEnhance® (Nanoprobes Inc., catalog number 2113) solution was prepared according to the manufacturer's specifications. Several drops of the solution were then applied to the surface of the sample from Example 6, and left there for 15 minutes before rinsing with water and drying with compressed $N_2$. Examples of AFM images obtained after this treatment are shown in **Figure 9(b)** and **Figure 10(b).**

Example 8: Control Experiment for Example 7

DNA (from calf thymus, Sigma-Aldrich, product number D-1501) was spin-stretched onto plasma-treated silicon as described in Example 2. This sample was treated with GoldEnhance® solution for 15 minutes, as described in Example 7. Two examples of AFM images obtained after this treatment are shown in **Figure 11.**

Example 9: Linker/Nucleic Acid Conjugate from Compound 3 and DNA

A small vial containing 13 nmoles of the anthraquinone derivative 3 dissolved in 20 µL of ethanol was mixed while 0.28 mL of the DNA in phosphate buffered saline solution from Example 6 was added dropwise (10 drops). The resulting clear solution was stored at 4°C for at least 24 hours. The conjugate was immobilized on $O_2$-plasma treated silicon and inspected by AFM. One of the images obtained is shown in **Figure 12(a).**

Example 10: Nanoparticle/Nucleic Acid Composite from the Conjugate from Example 9 and Phosphine-stabilised Gold Nanoparticles

Several drops of the solution of phosphine-stabilised gold nanoparticles were applied to the sample from Example 9 and left there for 1 hour before rinsing with water and drying with compressed $N_2$. One of the AFM images obtained afterwards is shown in **Figure 12(b).**

**Claims**

1. A linker molecule, comprising
   one or more nucleic acid binding group(s) and one or more nanoparticle binding group(s) which are connected covalently by a spacer group.

2. A linker molecule according to claim 1, wherein said nucleic acid binding group is selected from the group of agents comprising intercalating agents, groove-binding agents, alkylating agents, and combinations thereof.

3. A linker molecule according to claim 2, wherein the intercalating agent is selected from the group of compounds comprising acridines, anthraquinones, diazapyrenium derivatives, furanocoumarins (psoralens), naphthalene diimides, naphthalene monoimides, phenanthridines, porphyrins, and metal coordination complexes containing planar, aromatic ligands (metallointercalators).

4. A linker molecule according to claim 2, wherein the groove-binding agent is selected from the group of compounds comprising bis-benzamidines, bis-benzimidazoles, lexitropsins, perylene diimides, phenylbenzimidazoles, porphyrins, pyrrole oligopeptides and viologens.

5. A linker molecule according to claim 2, wherein the alkylating agent is selected from the group of compounds comprising aziridines, 2-chloroethane derivatives, epoxides, nitrogen mustards, sulfur mustards and metal coordination complexes having at least one leaving group ligand.

6. A linker molecule according to claim 5, wherein the metal coordination complexes that are alkylating agents are selected form the group of complexes comprising $Pt^{2+}$, $Pt^{4+}$, $Pd^{2+}$, $Ru^{2+}$, $Ru^{3+}$, $Rh^{1+}$, $Rh^{2+}$, and $Rh^{3+}$ having at least one ligand selected from the group comprising halide, water, di(alkyl)sulfoxide, nitrate, sulfate, carboxylate, substituted carboxylate, carbonate, phosphate, nitrite, sulfite, and hydroxide.

7. A linker molecule according to claim 1, wherein the nanoparticle binding group forms covalent bonds with surface ligands on the nanoparticle or displaces existing surface ligands on the nanoparticle, or combinations thereof.

8. A linker molecule according to claim 1 or 7, wherein the nanoparticle binding group comprises at least one covalent bond forming functional group selected from carboxylic acids and derivatives thereof, sulfonic acids and derivatives thereof, amines, alcohols, thiols, aldehydes, ketones, isocyanates, isothiocyanates, ethers, and halides.

9. A linker molecule according to claim 1 or 7, wherein the nanoparticle binding group comprises at least one metal-binding group selected from amines, phosphines, thiols, disulfides, dithiocarbamates, dithiophosphates, dithiophosphonates, thioethers, thiosulfates, and thioureas.

10. A method for the manufacture of a nanoparticle comprising conjugate, wherein a nanoparticle is combined with a linker molecule according to any of claims 1 to 9, forming a nanoparticle/linker conjugate.

11. A method for the manufacture of a nanoparticle-nucleic acid composite, wherein the nanoparticle/linker conjugate according to claim 10 is further reacted with a nucleic acid, forming a nanoparticle-nucleic acid composite.

12. A method for the manufacture of a nucleic acid comprising conjugate, wherein a nucleic acid molecule is reacted with a linker molecule according to any of claims 1 to 9, forming a nucleic acid/linker conjugate.

13. A method for the manufacture of a nanoparticle-nucleic acid composite, wherein the nucleic acid/linker conjugate according to claim 12 is further reacted with a nanoparticle, forming a nanoparticle-nucleic acid composite.

14. A method according to any of claims 11 to 13, **characterized in that** the nucleic acid is present dissolved in solution, preferably in an aqueous solution or immobilized on a substrate, preferably a non-metallic substrate or an electrode structure.

15. A method according to any of claims 11 to 14, **characterized in that** the nucleic acid is selected from the group comprising natural, modified, synthetic, and recombinant nucleic acids, DNA, RNA, PNA, CNA, oligonucleotides, oligonucleotides of DNA, oligonucleotides of RNA, primers, A-DNA, B-DNA, Z-DNA, polynucleotides of DNA, polynucleotides of RNA, T-junctions of nucleic acids, triplexes of nucleic acids, quadruplexes of nucleic acids, domains of non-nucleic acid polymer-nucleic acid blockcopolymers and combinations thereof.

16. A method according to any of claims 11 to 15, **characterized in that** the nucleic acid is double-stranded or single-stranded.

17. A method according to any of claims 10, 11 or 13, **characterized in that** the nanoparticle is catalytically active towards electroless plating

18. A method according to any of claims 10, 11, 13 or 17, **characterized in that** the nanoparticle contains a metal selected from the group comprising Fe, Co, Ni, Cu, Ru, Rh, Pd, Os, Ir, Pt, Ag, Au and combinations (e. g. alloys) of these metals.

19. A method according to any of claims 10, 11, 13, 17 or 18, **characterized in that** the nanoparticle's size is less than 10 nm.

20. A method according to claim 19, **characterized in that** the nanoparticle's size is between about 0.5 nm and about 3 nm.

21. A nanoparticle/linker conjugate or nucleic acid/linker conjugate obtainable according to a method of any of claims 10, 12, and 14 to 20.

22. A nanoparticle-nucleic acid composite obtainable according to a method of any of claims 11, 13, and 14 to 20.

23. A method for the manufacture of a nanowire, comprising electroless deposition of a metal onto a nanoparticle-nucleic acid composite according to claim 22.

24. A method according to claim 23, **characterized in that** the nanoparticle-nucleic acid composite is present dissolved in solution, preferably in an aqueous solution or immobilized on a substrate, preferably a non-metallic substrate or an electrode structure.

25. A method according to claim 23 or 24, **characterized in that** the metal deposited by electroless plating is selected from the group comprising Fe, Co, Ni, Cu, Ru, Rh, Pd, Os, Ir, Pt, Ag, Au and combinations (e. g. alloys) of these metals and magnetic and/or magnetized Fe, Co, Ni, and combinations (e. g. alloys) of these metals, and combinations (e. g. alloys) of these metals with B or P.

26. Nanowire obtainable by a method according to any of claims 23 to 25.

27. An electronic network comprising at least one nanowire according to claim 26.

28. An electronic circuit comprising an electronic network according to claim 27.

29. A junction between two or more wires of an electronic circuit, wherein each of the wires have an end segment proximal to the junction comprising a nanowire according to claim 26.

30. Use of the method according to any of claims 10 to 20 for selective metallisation of a nucleic acid.

Nucleic Acid
Binding Group(s)

Nanoparticle
Binding Group(s)

Spacer
Group

LINKER MOLECULE

Figure 1

**MAJOR GROOVE SIDE**

GUANINE          CYTOSINE          ADENINE          THYMINE

**MINOR GROOVE SIDE**

Figure 2

nucleic acid

+

linker molecule

nanoparticle

+

linker molecule

Linker/nucleic acid conjugate

Linker/nanoparticle conjugate

+ nanoparticle

+ nucleic acid

Nanoparticle-nucleic acid composite

Figure 3

INTERCALATING AGENT
(Anthraquinone)

GROOVE-BINDING AGENT
(Cationic porphyrin)

ALKYLATING AGENT
(Nitrogen mustard)

Figure 4

15

Figure 5

Figure 6

Figure 7

Figure 8

Figure 9

Figure 10

Figure 11

Figure 12

23

**European Patent Office**

**PARTIAL EUROPEAN SEARCH REPORT**

which under Rule 45 of the European Patent Convention shall be considered, for the purposes of subsequent proceedings, as the European search report

Application Number

EP 00 12 6966

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | I. ROMBECK, ET AL.: "Pd(gly-L-his-L-lys)Cl: soution structure and ternary complex formation with mono- and tertanucleotides" INORGANICA CHIMICA ACTA, vol. 273, no. 1,2, May 1998 (1998-05), pages 31-40, XP001010013 Lausanne, CH ISSN: 0020-1693 * compound 1 * | 1-3,5-9, 12,21 | C07C233/36 C07C323/60 C07H21/04 H01B1/12 H01L51/20 H01L51/30 G06N3/12 |
| X | J.-P. LAUSSAC, ET AL.: "NMR studies on binary and ternary Pd(II) complexes formed by the growth-modulating tripeptide glycylhistidyllysine and nucleotides" JOURNAL OF INORGANIC BIOCHEMISTRY, vol. 30, no. 3, 1987, pages 227-238, XP001010040 Elsevier Science Publishing, New York, NY, US * the whole document * | 1-3,5-9, 12,21 | |
| | -/-- | | |

**TECHNICAL FIELDS SEARCHED (Int.Cl.7)**

C07C
H01B
H01L
G06N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 9 July 2001 | English, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

EP 1 215 199 A1

**European Patent Office**

**INCOMPLETE SEARCH SHEET C**

Application Number

EP 00 12 6966

Claim(s) searched completely:
        none

Claim(s) searched incompletely:
        1-30

Reason for the limitation of the search:

Present claims 1-9 relate to compounds defined by reference to a desirable characteristic or property, namely their ability to bond to both nanoparticles and nucleic acids.

The claims cover all compounds having this characteristic or property, whereas the application provides support within the meaning of Article 84 EPC and/or disclosure within the meaning of Article 83 EPC for only a very limited number of such compounds.  In particular, the compounds are defined in terms of their being a solution to the problem being solved by the present invention, namely the provision of molecules which can link nanoparticles to nucleic acids.

Present claims 10-30 relate to methods using the linker molecules of claims 1-9 and the products of these processes.

In the present case, the claims so lack support, and the application so lacks disclosure, that a meaningful search over the whole of the claimed scope is impossible. Independent of the above reasoning, the claims also lack clarity (Article 84 EPC). An attempt is made to define the compound by reference to a result to be achieved. Again, this lack of clarity in the present case is such as to render a  meaningful search over the whole of the claimed scope impossible. Consequently, the search has been carried out for those parts of the claims which appear to be clear, supported and disclosed, namely those parts relating to the compounds illustrated in figures 4 and 5 , i.e. compounds 1-9.

**European Patent Office**    **PARTIAL EUROPEAN SEARCH REPORT**    Application Number

EP 00 12 6966

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | U. BEYER, ET AL.: "Synthesis and in vitro efficacy of transferrin conjugates of the anticancer drug chlorambucil" JOURNAL OF MEDICINAL CHEMISTRY, vol. 41, no. 15, 25 June 1998 (1998-06-25), pages 2701-2708, XP002170971 American Chemical Society, Washington, DC, US ISSN: 0022-2623 * page 2706, left-hand column, line 30 - line 36 * | 1,2,5,7,8 | |
| X | US 5 744 497 A (F. GIULIANI, ET AL.) 28 April 1998 (1998-04-28) * example 2 * | 1,2,5-9 | TECHNICAL FIELDS SEARCHED (Int.Cl.7) |
| A | EP 0 987 653 A (INTERNATIONAL BUSINESS MACHINES) 22 March 2000 (2000-03-22) * the whole document * | 1,10-30 | |
| D,A | W. POMPE, ET AL.: "Formation of metallic nanostructures on biomolecular templates" ZEITSCHRIFT FUR METALLKUNDE, vol. 90, no. 12, 1999, pages 1085-1091, XP000992857 Dr. Riederer Verlag GmbH, Stuttgart, DE ISSN: 0044-3093 * the whole document * | 1,10-30 | |
| D,A | J. RICHTER, ET AL.: "Nanoscale palladium metallisation of DNA" ADVANCED MATERIALS, vol. 12, no. 7, 2000, pages 507-510, XP000992850 VCH Verlagsgesellschaft, Weinheim, DE ISSN: 0935-9648 * the whole document * | 1,10-30 | |

-/--

EPO FORM 1503 03.82 (P04C10)

**European Patent**    PARTIAL EUROPEAN SEARCH REPORT    **Application Number**

**Office**

EP 00 12 6966

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | WO 99 29898 A (MAX PLANCK GESELLSCHAFT) 17 June 1999 (1999-06-17) * page 11, line 22 - line 28; examples * | 1,12,21 | |
| | | | **TECHNICAL FIELDS SEARCHED** (Int.Cl.7) |

EPO FORM 1503 03.82 (P04C10)

## ANNEX TO THE EUROPEAN SEARCH REPORT
## ON EUROPEAN PATENT APPLICATION NO.

EP 00 12 6966

This annex lists the patent family members relating to the patent documents cited in the above–mentioned European search report.
The members are as contained in the European Patent Office EDP file on
The European Patent Office is in no way liable for these particulars which are merely given for the purpose of information.

09-07-2001

| Patent document cited in search report | | Publication date | Patent family member(s) | | Publication date |
|---|---|---|---|---|---|
| US 5744497 | A | 28-04-1998 | IT | 1273391 B | 08-07-1997 |
| | | | AU | 682105 B | 18-09-1997 |
| | | | AU | 2110995 A | 23-10-1995 |
| | | | DE | 69507694 D | 18-03-1999 |
| | | | DE | 69507694 T | 22-07-1999 |
| | | | DK | 753000 T | 20-09-1999 |
| | | | EP | 0753000 A | 15-01-1997 |
| | | | FI | 963859 A | 27-09-1996 |
| | | | GR | 3030048 T | 30-07-1999 |
| | | | JP | 9506633 T | 30-06-1997 |
| | | | KR | 239787 B | 01-02-2000 |
| | | | NO | 963926 A | 19-09-1996 |
| | | | RU | 2130941 C | 27-05-1999 |
| | | | AT | 176478 T | 15-02-1999 |
| | | | CA | 2186731 A | 12-10-1995 |
| | | | CN | 1145624 A,B | 19-03-1997 |
| | | | CZ | 9602849 A | 16-04-1997 |
| | | | WO | 9526968 A | 12-10-1995 |
| | | | ES | 2129847 T | 16-06-1999 |
| | | | HU | 76293 A,B | 28-07-1997 |
| | | | JP | 2761297 B | 04-06-1998 |
| | | | NZ | 283143 A | 22-09-1997 |
| | | | ZA | 9502636 A | 02-01-1996 |
| EP 0987653 | A | 22-03-2000 | JP | 2000101167 A | 07-04-2000 |
| WO 9929898 | A | 17-06-1999 | EP | 1036202 A | 20-09-2000 |

EPO FORM P0459

For more details about this annex : see Official Journal of the European Patent Office, No. 12/82